# EUROPÄISCHE PATENTSCHRIFT

(11) **EP 1 757 254 B1**
(45) Veröffentlichungstag und Bekanntmachung des Hinweises auf die Patenterteilung: **14.07.2010**
(21) Anmeldenummer: 05018391.2
(22) Anmeldetag: 24.08.2005
(51) Int. Cl.: A61F 9/013

(54) **Mikrochirurgisches Schneidinstrument für refraktive ophthalmologische Behandlungen**
Microsurgical cutting instrument for use in refractive eye surgery
Instrument de coupe microsurgical pour traitement ophtalmologique réfractif.

(43) Veröffentlichungstag der Anmeldung: 28.02.2007
(73) Patentinhaber: WaveLight AG, 91058 Erlangen (DE)
(72) Erfinder: Donitzky, Christof, 90542 Eckental (DE); Thimm, Daniel, 90596 Schwanstetten (DE)
(74) Vertreter: von Hellfeld, Axel

(56) Entgegenhaltungen:
- EP-B1- 1 186 282
- US-A- 4 750 491
- US-A- 5 964 776
- US-B1- 6 569 174

## Beschreibung

Die Erfindung betrifft allgemein das Gebiet der refraktiven Behandlung des Auges, insbesondere des menschlichen Auges, zur Behebung von Fehlsichtigkeit.

Zur Behebung von Fehlsichtigkeiten niederer Ordnung wie etwa Myopie, Hyperopie, Astigmatismus, myoper Astigmatismus und hyperoper Astigmatismus haben sich unter anderem die Verfahren der sogenannten LASIK (LASer In situ Keratomileusis) und der sogenannten LASEK (LASer Epitheliale Keratomileusis) etabliert. Bei beiden Verfahren wird von der Oberfläche der Hornhaut ein Scheibchen (Flap) mit einem Durchmesser von beispielsweise etwa 8 bis 10 mm bis auf einen als Scharnier (Hinge) dienenden geringen Rest abgetrennt. Dieses Scheibchen wird zur Seite geklappt, wodurch das darunter liegende Hornhautmaterial für eine refraktiv wirkende Laserbehandlung zugänglich wird. Nach der Behandlung wird das Scheibchen wieder zurückgeklappt. Im Rahmen der Laserbehandlung, die üblicherweise mit einem Excimer-Laser bei einer Wellenlänge von beispielsweise 193 nm erfolgt, wird vom Hornhautstroma gemäß einem vorher bestimmten und ggf. während der Behandlung anzupassenden Ablationsprofil Material ablatiert (entfernt). Die hierdurch bewirkte Neuformung der Hornhaut verändert die Brechungseigenschaften der Hornhaut und damit des optischen Systems des Auges insgesamt. Weil wegen des zurückgeklappten Flaps keine nach außen offene Wunde bleibt, ist die Heilung regelmäßig relativ schnell.

Bei der LASIK wird der Flap mittels eines sogenannten Mikrokeratoms erzeugt, das den Flap von der Hornhautoberfläche abhobelt. Das Mikrokeratom weist einen Schneidkopf auf, der üblicherweise linear über die Hornhaut bewegt wird. Der Schneidkopf ist mit einer flachen Schneidklinge bestückt, die unter einem bestimmten Anstellwinkel zu einer Applanationsfläche des Schneidkopfs steht und um ein durch die gewünschte Flapdicke bestimmtes Maß über die Applanationsfläche vorsteht. Wird der Schneidkopf über die Hornhaut bewegt, schneidet und fährt die Schneidklinge in die Hornhaut ein und erzeugt dabei den Flap. Zusätzlich zum Vorschub des Schneidkopfs wird die Schneidklinge üblicherweise in laterale Oszillation versetzt.

Bei der LASIK-Methode war es lange Zeit gängig, den Flap mit einer Dicke von etwa 100 µm bis 200 µm zu erzeugen. Der Schnitt geht dabei durch das etwa 40 µm bis 60 µm dicke Hornhautepithel und die darunter liegende, etwa 8 µm bis 15 µm dicke Bowmanmembran direkt in das Stroma, dessen Dicke beispielsweise etwa 400 µm bis 500 µm beträgt.

In DE 103 17 972 B3 wird vorgeschlagen, den Schneidenwinkel und den Schneidenradius der Klingenschneide so einzustellen, dass die Klinge einerseits scharf genug ist, um das Hornhautepithel vollständig zu durchdringen, andererseits aber nicht scharf genug ist, um auch die Bowmanmembran zu durchdringen. Mit einer solchen Klinge soll erreicht werden, dass das Epithel sauber von der Bowmanmembran getrennt wird, ohne dass Epithelzellen zurückbleiben und ohne dass die Bowmanmembran verletzt wird. Der entstehende Flap ist somit ein reiner Epithelflap, der nur eine Dicke von etwa 50 µm hat.

Bei der LASIK-Metode besteht das Problem, dass die Hornhaut infolge ihrer Elastizität beim seitlichen Einfahren der Schneidklinge ausweichen kann. Die Folge kann ein unregelmäßiger Flaprand und eine nicht eindeutig reproduzierbare Flapgröße sein. Bei einer vergleichsweise stumpfen Schneide, wie sie in DE 103 17 972 B3 vorgeschlagen wird, kann dieses Problem besonders gravierend sein.

Bei der LASEK-Methode wird ebenfalls nur das Epithel von der Hornhaut gelöst. Die Flapgröße wird mittels eines sogenannten Mikrotrepans definiert, das ist ein Schneidelement mit einer entlang eines Kreisbogens verlaufenden Rundschneide, die sich nicht über einen geschlossenen Kreis erstreckt, sondern nur über einen Teilumfang eines Kreises von beispielsweise etwa 250 bis 300 Grad. Im verbleibenden Umfangsbereich ist ein Freiraum vorgesehen. Der Trepan wird auf das Auge aufgesetzt und dringt unter Hin- und Herdrehen um einen begrenzten kleinen Drehwinkel von beispielsweise etwa 10 Grad mit seiner Rundschneide in die Hornhaut ein. Es entsteht ein Rundschnitt im Epithel, dessen Länge gleich derjenigen der Rundschneide plus den Drehwinkel des Trepans ist. Der nicht eingeschnittene Bereich bildet das Scharnier des Flaps. Für den Schnitt wird der Trepan in einem Führungskörper mit einer zylindrischen Aufnahmeöffnung geführt, in die der Trepan eingesetzt wird.

Nach dem Schnitt mit dem Trepan wird mittels einer alkoholischen Lösung, die in einen auf das Auge aufgesetzten Zylinder geträufelt wird, das Epithelgewebe aufgeweicht, bis der Flap mit einem Schaber oder Spatel von der Bowmanmembran gelöst und zur Seite geschoben werden kann. Die Verwendung der Alkohollösung ist allerdings mit Nachteilen verbunden, da sie die Zellen der zwischen Epithel und Bowmanmembran liegenden Basalmembran abtöten kann. Dies verlangsamt den Wundverschluss, da neue Epithelzellen nur langsam einwachsen können.

Schneidinstrumente für LASIK und LASEK weisen generell eine auf das Auge aufzusetzende Instrumentengrundkörpereinheit auf, welche relativ zu dem Auge fixierbar ist. Zur Fixierung am Auge ist es bekannt, die Instrumentengrundkörpereinheit mit einem Saugring auszuführen, der auf den Limbus aufgesetzt wird und sich mittels Vakuum an diesen ansaugt. Hierzu weist die Instrumentengrundkörpereinheit einen Evakuierungsanschluss auf, der über ein Schlauchleitungssystem an eine externe Vakuumpumpe anschließbar ist und über ein in der Instrumentengrundkörpereinheit gebildetes Evakuierungswegssystem mit einer an der augenzugewandten Unterseite des Saugrings vorgesehenen ringförmigen Evakuierungsnut verbunden ist.

Bisherige Schneidinstrumente sind entweder speziell für die LASIK oder speziell für die LASEK ausgestaltet.

US 4,750,491 offenbart einen Trepan mit auswechselbaren Schneideinsätzen. Die Schneideinsätze gestatten jeweils die Einbringung eines Rundschnitts in die Kornea in unterschiedlicher Winkellage. Insgesamt kann so eine ringförmig verlaufende Nut in die Korneaoberfläche eingearbeitet werden.

US 6,569,174 B1 offenbart ein Mikrokeratom mit einem flachen Klingenblatt, welches der Erzeugung eines Korneaflaps dient. Das Klingenblatt befindet sich knapp oberhalb einer Blende, deren Blendenöffnung etwas größer als die gewünschte Größe des zu erzeugenden Flaps ist. Oberhalb des Klingenblatts befindet sich eine transparente Applanationsplatte, welche den durch die Blendenöffnung hindurchragenden Teil der Hornhaut flach drückt, so dass vor dem Klingenblatt ein im wesentlichen gleichmäßig dicker Hornhautpfropfen steht. Dies soll eine gleichmäßige Dicke des zu schneidenden Flaps gewährleisten. Am Rand der Blendenöffnung befindet sich eine nach unten ragenden Ringschneide, mittels welcher kreisförmige, elliptische, ovale oder andersförmige Ringschnitte in die Hornhaut eingebracht werden können. Diese Ringschnitte dienen nach den Angaben in dem Dokument der zirkulären Keratotomie.

Aufgabe der Erfindung ist es, ein mikrochirurgisches Schneidinstrument bereitzustellen, mit dem sich verbesserte Behandlungsergebnisse als bei bisherigen LASEK und LASIK-Methoden erzielen lassen.

Zur Lösung dieser Aufgabe ist erfindungsgemäß ein mikrochirurgisches Schneidinstrument nach Anspruch 1 vorgeseben. Das Schneidinstrument ist für refraktive ophthalmologische Behandlungen vorgesehen und umfasst eine auf das Auge aufzusetzende, relativ zu diesem fixierbare Instrumentengrundkörpereinheit, wobei der Instrumentengrundkörpereinheit Führungshaltemittel zugeordnet sind, welche zur relativ zur Instrumentengrundkörpereinheit beweglich geführten Halterung von mindestens zwei Schneideinheiten mit unterschiedlicher Schneidengeometrie bestimmt und ausgebildet sind. Insbesondere können die Führungshaltemittel mindestens eine erste Führungsformation zur Führung einer ersten Schneideinheit und mindestens eine von der ersten Führungsformation verschiedene zweite Führungsformation zur Führung einer zweiten Schneideinheit umfassen.

Mit einem erfindungsgemäßen Schneidinstrument lassen sich Vorteile der herkömmlichen LASEK und LASIK-Techniken in einem einzigen Gerät vereinen. So ist die Instrumentengrundkörpereinheit mit einer ersten Schneideinheit bestückt oder bestückbar, welche es gestattet, einen Schnitt zu setzen, der nur den Flaprand festlegt, ohne dabei unter das Epithel zu fahren und dieses abzuheben. Die Instrumentengrundkörpereinheit ist ferner mit einer zweiten Schneideinheit bestückt oder bestückbar, welche es gestattet, in den durch die erste Schneideinheit gesetzten Schnitt einzufahren und den Flap vom darunter liegenden Hornhautmaterial abzulösen. Es kann so auf die Verwendung einer Alkohollösung verzichtet werden, gleichzeitig ist es möglich, Flaps reproduzierbarer Größe mit sauberem, gleichmäßigem Rand zu erzeugen. Zweckmäßigerweise weist die erste Schneideinheit eine Rundschneide auf, wobei die mindestens eine erste Führungsformation zur rotatorischen Führung der ersten Schneideinheit ausgebildet ist, während die zweite Schneideinheit eine geradlinige Schneide aufweist und die mindestens eine zweite Führungsformation zur wenigstens näherungsweise linearen Führung der zweiten Schneideinheit ausgebildet ist.

Da mit dem ersten Schnitt der Flaprand eindeutig festgelegt wird, kann die Schneide der zweiten Schneideinheit vergleichsweise stumpf ausgeführt werden. Insbesondere kann sie so stumpf ausgeführt werden, dass im wesentlichen keine Gefahr besteht, dass sie in die Bowmanmembran einschneidet und dadurch in das Hornhautstroma gelangt. Es muss lediglich eine solche Schärfe der Schneide der zweiten Schneideinheit gewährleistet sein, dass sie das Epithel von der Bowmanmembran ablösen kann. Im Unterschied zu der in DE 103 17 972 B3 gelehrten Methodik muss sich die Schneide der zweiten Schneideinheit nicht selbst von außen in das Epithel einschneiden. Sie kann stattdessen den von der ersten Schneideinheit zuvor eingebrachten Rundschnitt als "Eintritt" nutzen und wird dann in dem Rundschnitt sozusagen geführt.

Die Führungshaltemittel können einen von der Instrumentengrundkörpereinheit gesonderten, jedoch an diesem positionierbaren Führungskörper umfassen, an dem die mindestens eine erste Führungsformation ausgebildet ist, während die mindestens eine zweite Führungsformation an der Instrumentengrundkörpereinheit ausgebildet ist. Für den Schneidbetrieb der zweiten Schneideinheit kann dabei der Führungskörper von der Instrumentengrundkörpereinheit abzunehmen sein.

Der Führungskörper weist vorzugsweise eine näherungsweise zylindrische Führungsaufnahmeöffnung auf, in der die erste Schneideinheit um die Zylinderachse drehbar aufnehmbar ist. Dazu weisen der Führungskörper und die Instrumentengrundkörpereinheit vorteilhafterweise zusammenwirkende Verdrehsicherungsmittel auf, welche den Führungskörper gegen Verdrehung relativ zur Instrumentengrundkörpereinheit um die Zylinderachse sichern.

Die Schneide der zweiten Schneideinheit kann von einer flachen Schneidklinge gebildet sein, wobei dann ein Anstellwinkel der Schneidklinge zwischen 18 und 32 Grad, vorzugsweise zwischen 21 und 28 Grad, höchstvorzugsweise von etwa 25 Grad gegenüber der Führungsrichtung der zweiten Schneideinheit bevorzugt wird.

Das erfindungsgemäße Schneidinstrument erlaubt die Erzeugung eines Hornhautflaps am Auge durch:
- Einbringen eines Rundschnitts in die Hornhaut mittels einer eine Rundschneide aufweisenden ersten Schneideinheit,
- Einfahren einer geradlinigen Schneide einer zweiten Schneideinheit in die Hornhaut ausgehend von dem Rundschnitt zur Erzeugung des Flaps.

Mit dem erfindungsgemäßen Schneidinstrument können ohne zwischenzeitliches Abnehmen der Instrumentengrundkörpereinheit vom Auge nacheinander die erste und die zweite Schneideinheit zum Einsatz gebracht werden.

Der Rundschnitt wird bevorzugt nur so tief in die Hornhaut eingebracht, dass ohne Verletzung der Bowmanmembran ein ca. 50 µm dicker Epithelflap von der Hornhautoberfläche abgelöst werden kann. Wie zuvor erwähnt, ist hierzu die Schärfe der geradlinigen Schneide bevorzugt derart bemessen, dass beim Einfahren der geradlinigen Schneide in die Hornhaut eine Verletzung die Bowmanmembran im wesentlichen nicht zu befürchten ist.

Die zweite Schneideinheit besitzt bevorzugt ein definiert stumpfes Schneidelement mit geradliniger, im Querschnitt rundlicher Schneidkante mit einem Rundungsradius zwischen 1 und 10 µm, vorzugsweise zwischen 3 und 8 µm, höchstvorzugsweise zwischen 4 und 6 µm. Außerdem besitzt das Schneidelement in einem unmittelbar an die Schneidkante anschließenden ersten Schneidenbereich einen Schneidenwinkel zwischen 15 und 22 Grad, vorzugsweise zwischen 16 und 20 Grad, höchstvorzugsweise von etwa 18 Grad.

In einem an den ersten Schneidenbereich anschließenden zweiten Schneidenbereich kann das Schneidelement einen Schneidenwinkel zwischen 10 und 18 Grad, vorzugsweise zwischen 11 und 16 Grad, höchstvorzugsweise von etwa 13 Grad besitzen, wobei der Schneidenwinkel des zweiten Schneidenbereichs kleiner als der Schneidenwinkel des ersten Schneidenbereichs ist. Gewünschtenfalls kann das Schneidelement ferner in einem an den zweiten Schneidenbereich anschließenden dritten Schneidenbereich einen Schneidenwinkel zwischen 7 und 15 Grad, vorzugsweise zwischen 8 und 12 Grad, höchstvorzugsweise von etwa 9 Grad besitzen, wobei der Schneidenwinkel des dritten Schneidenbereichs kleiner als der Schneidenwinkel des zweiten Schneidenbereichs ist.

Die Erfindung wird nachfolgend anhand der beigefügten Zeichnungen näher erläutert. Es stellen dar:
- Figur 1: perspektivisch ein mikrochirurgisches Schneidinstrument - bestückt mit einem Trepan - gemäß einem Ausführungsbeispiel der Erfindung,
- Figur 2: einen Schnitt durch das Schneidinstrument der Figur 1 in einem Zustand, in dem dieses auf ein Auge aufgesetzt ist,
- Figur 3: perspektivisch einen mit dem Schneidinstrument der Figur 1 verwendbaren Schneidkopf,
- Figur 4: in vergrößerter Schnittdarstellung den Bereich des Schneidkopfs der Figur 3, in dem die Schneidklingeneinheit angeordnet ist,
- Figur 5: eine perspektivische Ansicht der mit dem Schneidkopf der Figur 3 verwendeten Schneidklingeneinheit,
- Figuren 6 und 7: stark schematisiert zwei aufeinanderfolgende Phasen des erfindungsgemäßen Verfahrens zur refraktiven Behandlung eines Auges,
- Figur 8: perspektivisch das Schneidinstrument der Fig. 1 und 2 - mit geringfügigen Abweichungen - in einem operationsbereiten Zustand, in dem es mit einem Trepan und einem Schneidkopf bestückt ist, und
- Figur 9: vergrößert einen schneidkantennahen Teil einer mit dem Schneidkopf der Fig. 4 und 5 verwendbaren Schneidklinge.

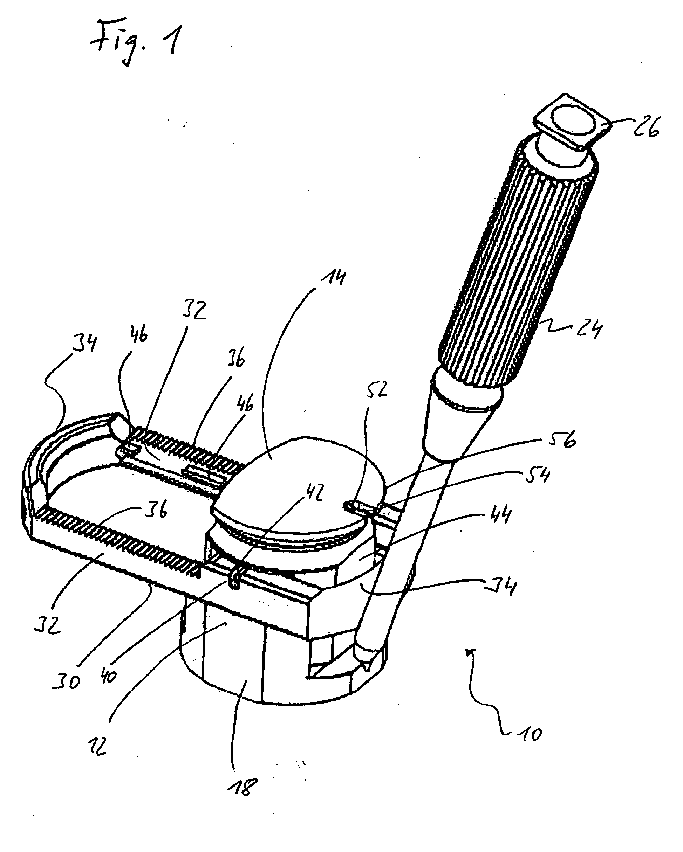

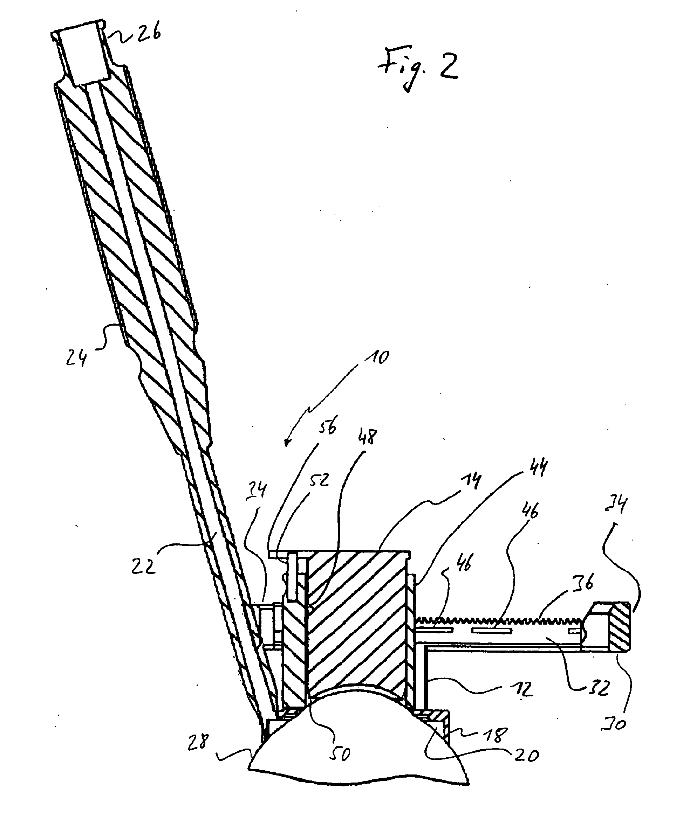

Es wird zunächst auf die Figuren 1 und 2 verwiesen. Das dort dargestellte mikrochirurgische Schneidinstrument ist allgemein mit 10 bezeichnet. Es ist für mikrochirurgische Behandlungen am Auge, insbesondere menschlichen Auge, vorgesehen, die dem Ziel einer Neuformung der Hornhaut zur Behebung von Fehlsichtigkeiten dienen. Im Rahmen einer solchen Behandlung wird zunächst mittels des Schneidinstruments 10 ein Epithelflap von der Hornhautoberfläche abgelöst. Nachdem der Epithelflap zur Seite geklappt ist, wird sodann eine Behandlung mit einem refraktiv wirkenden Laser durchgeführt, wobei Material vom Hornhautstroma ablatiert wird. Nach beendeter Laserbehandlung wird der Epithelflap zurückgeklappt.

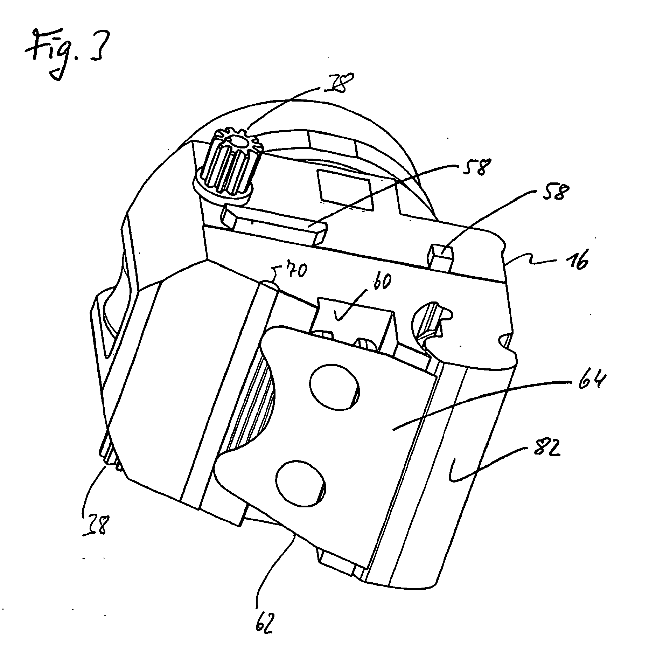

Das Schneidinstrument 10 weist einen beispielsweise aus Edelstahl gefertigten Instrumentengrundkörper 12 auf, an dem wechselweise ein eine erste Schneideinheit bildender Trepan 14 und ein eine zweite Schneideinheit bildender Schneidkopf 16 (Figur 3) anbringbar sind. Der Instrumentengrundkörper bildet einen Saugring 18 mit einer Evakuierungsnut 20, welche Teil eines Evakuierungswegsystems 22 ist, das sich durch ein längliches Griffstück 24 des Schneidinstruments 10 bis zu einem Evakuierungsanschluss 26 erstreckt. An den Evakuierungsanschluss 26 ist in nicht näher dargestellter Weise eine Schlauchleitung anschließbar, welche mit einer in einer Basisstation einer Einrichtung zur refraktiven ophthalmologischen Behandlung untergebrachten Vakuumpumpe verbindbar ist. Zur Fixierung des Instrumentengrundkörpers 12 am Auge wird der Saugring 18 auf den Limbus (Hornhautrand) aufgesetzt und durch Anlegen von Vakuum an das Evakuierungswegsystem 22 ein Saugeffekt erzeugt, der den Saugring 18 an das in Figur 2 mit 28 bezeichnete Auge ansaugt.

Der Instrumentengrundkörper 12 weist ferner einen mit dem Saugring 18 insbesondere einstückig verbundenen Führungs- und Halterungsrahmen 30 auf, welcher aus zwei im Abstand parallel zueinander sich erstreckenden, geradlinigen Längsstreben 32 und zwei die Längsstreben 32 an ihren Enden miteinander verbindenden, bogenartig gekrümmten Verbindungsstücken 34 besteht. Auf ihrer augenabgewandten Oberseite sind die Längsstreben 32 auf einem Teil ihrer Längserstreckung mit einer Verzahnung 36 ausgeführt, welche zum Verzahnungseingriff mit Antriebsritzeln 38 (Figur 3) des Schneidkopfes 16 dient. Im verzahnungsfreien Bereich ihrer augenabgewandten Oberseite weisen die Längsstreben 32 ferner jeweils eine Einkerbung 40 auf, welche zur Aufnahme eines Positionierzapfens 42 einer Führungshülse 44 für den Trepan 14 dient. Des weiteren sind an den einander zugewandten Innenseiten der Längsstreben 32 Rippenstücke 46 ausgebildet, welche der linear beweglichen Führung des Schneidkopfs 16 dienen.

Die Führungshülse 44 ist ein von dem Instrumentengrundkörper 12 gesondertes Bauteil und dient zur drehbeweglichen Führung des Trepans 14. Sie wird zwischen die beiden Längsstreben 32 in einem Bereich über dem Saugring 18 eingesetzt und wird durch den Eingriff ihrer Positionierzapfen 42 in die Einkerbungen 40 einerseits nach unten (bezogen auf die Sichtweise in den Figuren 1 und 2) abgestützt und andererseits gegen Verdrehung relativ zu dem Instrumentengrundkörper 12 gesichert. Sie weist eine kreiszylindrische Innenumfangsfläche 48 auf, welche als Führungsfläche zur drehbeweglichen Führung des Trepans 14 dient. Der Trepan 14 weist, wie in Figur 2 angedeutet, an seiner unteren, also in Gebrauchsstellung dem Auge 28 zugewandten Seite eine kreisbogenförmig sich erstreckende Schneide 50 auf, welche sich in Umfangsrichtung beispielsweise über etwa 250 bis 300° erstreckt. Auf dem Rest des Kreisumfangs ist die Schneide 50 des Trepans 14 in an sich bekannter Weise ausgespart, sodass in dem ausgesparten Bereich kein Schneidkontakt mit der Hornhaut des Auges 28 stattfindet. Aufgrund Ihrer Erstreckung längs eines Kreisbogens wird die Schneide 50 hier auch als Rundschneide bezeichnet. Die Rundheit der Schneide 50 stellt eine erste Schneidengeometrie im Sinne der Erfindung dar. Es ist darauf hinzuweisen, dass der Begriff "rund" im Zusammenhang mit der Schneide 50 sich nicht auf den Schneidenquerschnitt, (d.h. den Schneidenradius) bezieht, sondern lediglich - wie soeben erwähnt - auf den kreisbogenförmigen Verlauf der Schneide 50.

In der Darstellung der Figur 2 ist der Trepan 14 als ein Vollmaterialkörper gezeigt. Es versteht sich, dass alternativ ein rohr- oder hülsenförmiger Körper als Trepan verwendet werden kann.

Der Trepan 14 ist um einen begrenzten Drehwinkel relativ zu der Führungshülse 44 verdrehbar. Zur Drehwinkelbegrenzung dient ein im Beispielfall der Figuren 1 und 2 an der Führungshülse 44 vorgesehener, axial vorstehender Zapfen 52, welcher in eine Aussparung 54 eingreift, welche in einen radial (bezogen auf die Achse der Führungshülse 44) abstehenden Bund 56 des Trepans 14 eingeformt ist. Die Umfangsweite der Ausnehmung 54 bestimmt den Relativdrehwinkel, um den der Trepan 14 gegenüber der Führungshülse 44 verdrehbar ist. Es versteht sich, dass bei einer abgewandelten Ausführungsform die Führungshülse 44 eine drehwinkelbegrenzende Ausnehmung haben kann, in die ein Zapfen des Trepans 14 eingreifen kann. Es sind auch andere Ausgestaltungen von Anschlagmitteln möglich, die zwischen der Führungshülse 44 und dem Trepan 14 wirksam sind und für eine Drehwinkelbegrenzung sorgen.

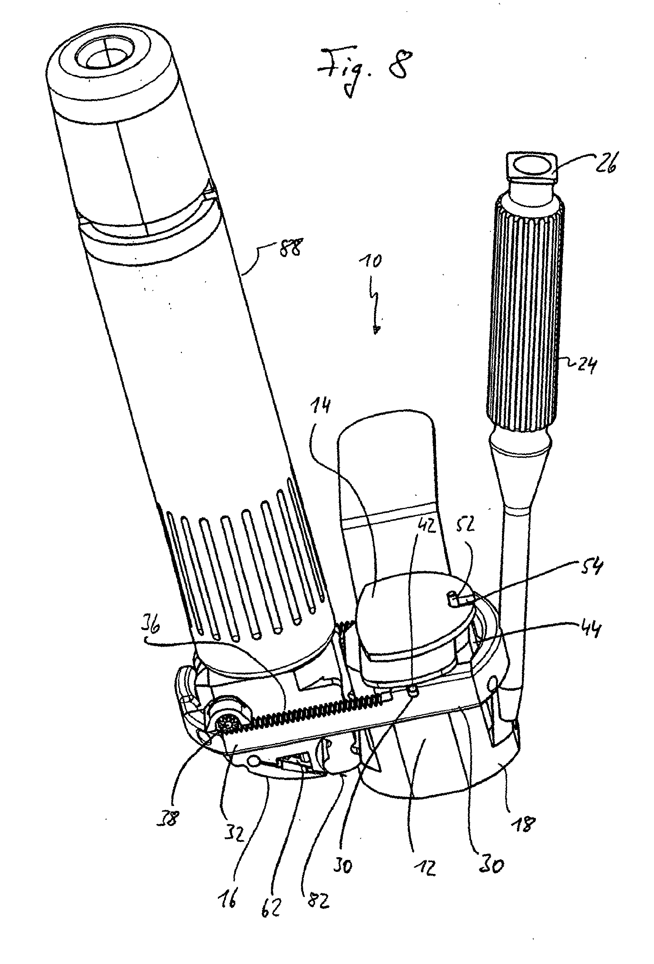

Der Schneidkopf 16 und die Führungshülse 44 mit darin eingesetztem Trepan 14 können gleichzeitig an dem Instrumentengrundkörper 12 angeordnet sein. Dieser Zustand des Schneidinstruments ist in Fig. 8 gezeigt. Es kann demnach schon vor der Operation der Instrumentengrundkörper 12 mit beiden Schneideinheiten, also Trepan 14 und Schneidkopf 16, bestückt werden. Für die Ingebrauchnahme des Trepans 14 kann der Schneidkopf 16 an dem Instrumentengrundkörper 16 bleiben; der Schneidkopf 16 stört hierbei nicht. Für die Ingebrauchnahme des Schneidkopfs 16 ist allerdings zuvor die Führungshülse 44 von dem Instrumentengrundkörper 12 abzunehmen. Andernfalls könnte der Schneidkopf 16 nicht nach vorne über das Auge bewegt werden.

Der Schneidkopf 16 wird an dem Instrumentengrundkörper 12 montiert, indem er im hinteren Bereich des Führungs- und Halterungsrahmens 30 zwischen die Längsstreben 32 eingesetzt wird. Wie in Figur 3 gezeigt, weist auch der Schneidkopf 16 auf seinen beiden Lateralseiten Rippenstücke 58 auf, die dann, wenn der Schneidkopf 16 ordnungsgemäß an dem Instrumentengrundkörper 12 angebracht ist, die Rippenstücke 46 des Rahmens 30 untergreifen und sich an diesen entlangbewegen. Die Antriebsritzel 38 des Schneidkopfs 16 greifen in diesem ordnungsgemäß eingesetzten Zustand in die Verzahnungen 36 der Längsstreben 32 kämmend ein. Der Schneidkopf 16 ist in nicht näher dargestellter Weise an elektromotorische Antriebsmittel ankoppelbar, durch welche die Antriebsritzel 38 antreibbar sind. Bei Antrieb der Antriebsritzel 38 bewegt sich der Schneidkopf 16 demzufolge längs der Längsstreben 32, wobei eine in einer Aufnahmetasche 60 des Schneidkopfs 16 aufgenommene Schneidklingeneinheit 62 über die Hornhaut des zu behandelnden Auges fährt und dabei einen Flap von der Hornhaut ablöst.

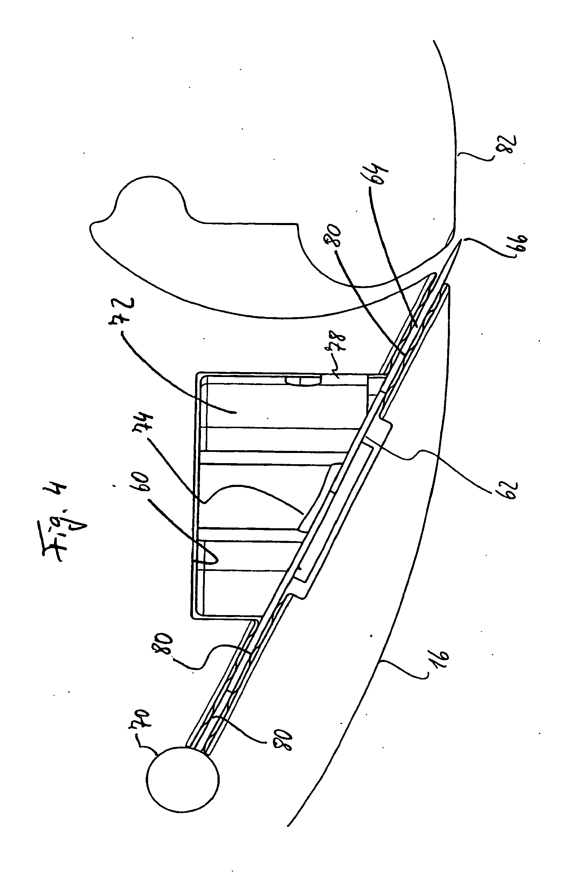

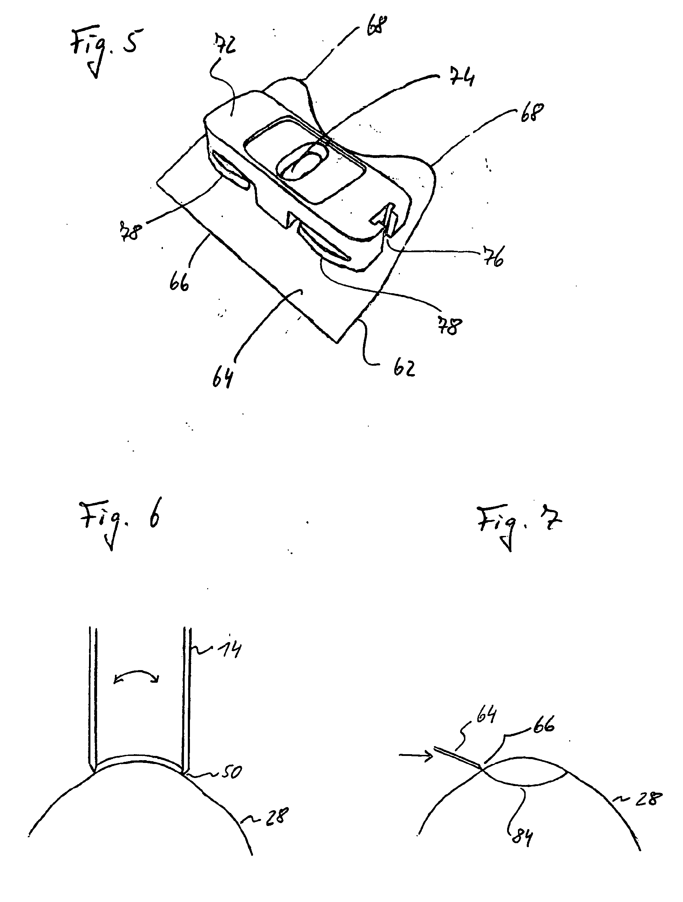

Die Schneidklingeneinheit 62 ist gut in Figur 5 erkennbar. Sie umfasst eine beispielsweise aus Edelstahl gefertigte, flache Schneidklinge 64 mit einer Schneidkante 66 an einem vorderen Klingenrand und mehreren (hier zwei) im Abstand voneinander vorgesehenen Anlageabschnitten 68 an einem hinteren Klingenrand. Zwischen den Anlageabschnitten 68 ist der hintere Klingenrand zurückversetzt. Im dargestellten Beispielfall sind die Anlageabschnitte 68 rundlich ausgeführt; sie dienen zur Abstützung an einer im Schneidkopf 16 vorgesehenen konvexen Widerlagerfläche, welche beispielsweise von einem Widerlagerstab 70 (Figuren 3, 4) gebildet sein kann. Zwischen den Anlageabschnitten 68 der Schneidklinge 64 und dem Widerlagerstab 70 besteht näherungsweise Punktkontakt, was für geringe Reibung und entsprechend geringen Verschleiß sorgt.

Die Schneidklingeneinheit 62 weist ferner einen Aufsatz 72 auf einer der flachen Klingenseiten auf, welcher beispielsweise aus Kunststoff hergestellt ist und fest mit der Schneidklinge 64 verbunden ist. Der Aufsatz 72 ist auf seiner von der Schneidklinge 64 abgelegenen Oberseite mit einer länglichen Vertiefung 74 ausgeführt, in welche im Betrieb des Schneidkopfs 16 ein nicht näher dargestellter Exzenterzapfen einer Antriebswelle der erwähnten elektromotorischen Antriebsmittel eingreift. Hierdurch wird die Schneidklingeneinheit 62 in laterale Oszillation versetzt, die dem durch den Eingriff der Antriebsritzel 38 mit den Verzahnungen 36 bewirkten Vorschub des Schneidkopfs 16 überlagert ist.

Auf mindestens einer seiner lateralen Seiten weist der Aufsatz 72 ferner eine hinterschnittene T-Nut 76 auf, welche der Ankopplung eines nicht näher dargestellten Schiebers dient, mit dem die Schneidklingeneinheit 62 in die Aufnahmetasche 60 des Schneidkopfs 16 geschoben und nach Gebrauch wieder herausgezogen werden kann. Die Nut 76 ermöglicht so eine einfache Handhabung der Schneidklingeneinheit 62.

Der Aufsatz 72 trägt schließlich noch Federelemente 78, welche auf der vom hinteren Klingenrand abgewandten Seite des Aufsatzes 72 angeordnet sind. Wenn die Schneidklingeneinheit 62 in die Aufnahmetasche 60 des Schneidkopfs 16 eingesetzt ist, wirken die Federelemente 78 derart mit einer Begrenzungsfläche der Aufnahmetasche 60 zusammen, dass sich eine Vorspannung einstellt, durch die die Schneidklingeneinheit 62 gegen die rückwärtige Widerlagerfläche des Schneidkopfs 16 gedrückt wird. Hierdurch wird eine präzise Positionierung und Ausrichtung der Schneidklingeneinheit 62 in der Aufnahmetasche 60 erzielt.

Wie gut in Figur 4 erkennbar, ist die Aufnahmetasche 60, die zu mindestens einer lateralen Seite des Schneidkopfs 60 offen ist, mit einem querschnittserweiterten Mittelabschnitt zur Aufnahme des Aufsatzes 72 und zwei davor bzw. dahinter liegenden schlitzartig verengten Abschnitten ausgeführt, in denen Klingenführungsformationen 80 zur beidseitigen Führung der Schneidklinge 64 vorgesehen sind. Die Klingenführungsformationen 80 können beispielsweise von Führungsrippen gebildet sein, welche sich in Klingenquerrichtung erstrecken.

Der Schneidkopf 16 besitzt eine in Vorschubrichtung vor der Schneidkante 66 der Schneidklinge 64 angeordnete Applanationsfläche 82, welche im Gebrauch des Schneidkopfs 16 gegen die Hornhautoberfläche drückt. Die Schneidkante 66, die eine geradlinige Schneide und somit eine weitere Schneidengeometrie im Sinne der Erfindung bildet, steht etwas über die Applanationsfläche 82 vor, um bei Antrieb des Schneidkopfs 16 in die Hornhaut eindringen zu können.

Die die Schneidwirkung der Schneidklinge 64 beeinflussenden Parameter sind so eingestellt, dass die Schneidklinge 64 die Bowmanmembran und somit das Hornhautstroma nicht verletzt. Dies wird insbesondere durch eine gewisse Stumpfheit der Schneidkante 66 erreicht, die so gewählt ist, dass die Schneidklinge 64 zwar das Hornhautepithel von der Bowmanmembran ablösen kann, diese Membran jedoch nicht durchdringen kann. Darüber hinaus kann der Anstellwinkel der Schneidklinge 64 gegenüber der Applanationsfläche 82 bzw. gegenüber der Vorschubrichtung des Schneidkopfs 16 Einfluss auf die Schneidwirkung haben. Günstige Ergebnisse haben sich bei einem Wert des Anstellwinkels von etwa 25 Grad eingestellt. Auch der Überstand der Schneidkante 66 über die Applanationsfläche 82 ist zweckmäßigerweise so gering eingestellt, dass er im wesentlichen der gewünschten Flapdicke entspricht, also etwa der Dicke des Hornhautepithels (typischerweise etwa 50 bis 55 µm).

Aus Gründen der Übersichtlichkeit ist der in Figur 4 unterhalb der Schneidklinge 64 liegende Teil des Schneidkopfs 16 in Figur 3 weggelassen, sodass dort die Schneidklinge 64 vollständig von unten sichtbar ist.

Die Figuren 6 und 7 zeigen schematisch die beiden wesentlichen Phasen zur Erzeugung eines Epithelflaps mit dem Schneidinstrument 10. Zunächst wird gemäß Figur 6 mit dem Trepan 14 ein Rundschnitt in das Hornhautepithel des Auges 28 eingebracht. Hierzu wird der Trepan 14 auf die Hornhautoberfläche aufgesetzt und leicht hin und her gedreht. Hierbei dringt die Schneide 50 des Trepans 14 in das Epithel ein. Die Eindringtiefe des Trepans 14 kann beispielsweise durch nicht näher dargestellte Anschlagmittel festgelegt sein, welche zwischen dem Trepan 14 und der Führungshülse 44 in axialer Richtung wirksam sind.

Der so mit dem Trepan 14 eingebrachte Rundschnitt ist in Figur 7 mit 84 bezeichnet. In einer anschließenden Phase des Verfahrens zur Erzeugung des Epithelflaps kommt der Schneidkopf 16 mit der Schneidklinge 64 zum Einsatz. Während des Ausbaus des Trepans 14 und der Führungshülse 44 bleibt der Instrumentengrundkörper 12 am Auge 28 festgesaugt. Durch Antrieb des Schneidkopfes 16 wird die Schneidklinge 64 linear über das Auge 28 bewegt. Sie fährt hierbei in den zuvor eingebrachten Rundschnitt 84 ein und hebt dann das Epithel unter Erzeugung des gewünschten Flaps von der Bowmanmembran ab. Es versteht sich, dass hierzu eine geeignete Abstimmung der Montagepositionen der Führungshülse 44 und des Trepans 14 einerseits und des Schneidkopfs 16 andererseits an dem Instrumentengrundkörper 12 erforderlich ist, damit die Schneidklinge 64 präzise auf den vom Trepan 14 bewirkten Rundschnitt 84 trifft und diesen sozusagen als "Eintritt" in das Hornhautepithel nutzen kann.

Bei dem soweit beschriebenen Ausführungsbeispiel bilden der Rahmen 30 und die Führungshülse 44 Führungshaltemittel im Sinne der Erfindung. Es versteht sich, dass die Führungshaltemittel keineswegs auf eine derartige Ausbildung, wie sie in den Figuren 1 und 2 gezeigt ist, beschränkt sind. Es sind ohne weiteres vielfältige Abwandlungen vorstellbar. Der Begriff Führungshaltemittel ist deshalb ganz allgemein so zu verstehen, dass er jegliche Strukturen umfasst , die dazu geeignet sind, verschiedene Schneideinheiten mit unterschiedlicher Schneidengeometrie an einem Instrumentengrundkörper eines erfindungsgemäßen mikrochirurgischen Schneidinstruments zu halten und beweglich zu führen.

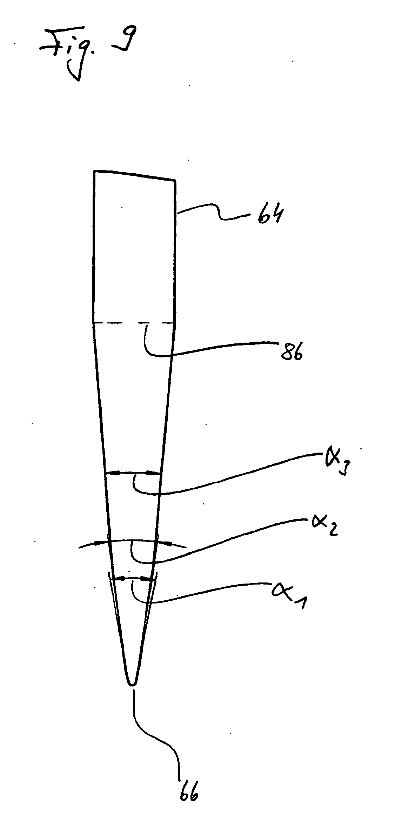

Hinsichtlich einer bevorzugten Schneidengeometrie der Schneidklinge 64 wird nun auf Fig. 9 verwiesen. Diese zeigt im Schnitt einen Ausschnitt der Schneidklinge 64 im Bereich der Schneidkante 66. Man erkennt, dass die Schneidkante 66 rundlich ausgeführt ist. Der Rundungsradius an der Schneidkante 66 beträgt vorzugsweise etwa 5 µm. Die Schneidkante 66 befindet sich am Ende eines Klingenabschnitts, in dem die gegenüberliegenden Klingenseiten noch nicht parallel zueinander verlaufen, sondern einen Winkel zwischen sich einschließen. Dieser Winkel wird hier Schneidenwinkel genannt. Der Schneidenwinkel variiert bei dem hier gezeigten Ausführungsbeispiel. In einem unmittelbar an die Schneidkante 66 anschließenden ersten Schneidenbereich beträgt der Schneidenwinkel - bezeichnet mit α₁ - etwa 18 Grad, wobei er von diesem Wert nach unten vorzugsweise nur wenig, am besten gar nicht abweicht, nach oben jedoch bis zu 2 Grad abweichen kann. Der erste Schneidenbereich kann sich beispielsweise über eine Länge von etwa 0,10 bis 0,15 mm erstrecken.

In einem anschließenden zweiten Schneidenbereich ist der Schneidenwinkel - dort bezeichnet mit a₂ - kleiner als im ersten Schneidenbereich und beträgt etwa 13 Grad, wobei eine Abweichung von etwa 4 Grad nach oben zugelassen ist, nach unten jedoch vorzugsweise keine Abweichung zugelassen ist. Schließlich ist noch ein dritter Schneidenbereich vorgesehen, in dem der Schneidenwinkel - bezeichnet mit α₃ - erneut kleiner als in den vorherigen Schneidenbereichen ist und etwa 9 Grad mit einer zulässigen Abweichung von etwa 3 Grad nach oben beträgt. Nach unten sollte der Schneidenwinkel a₃ nicht kleiner als dieser Wert sein. Insgesamt kann der Klingenabschnitt von der Schneidkante 66 bis zu der Stelle, an der die gegenüberliegenden Klingenseiten in Parallelität übergehen (angedeutet durch eine Linie 86 in Fig. 9), etwa 0,45 bis 0,50 mm lang sein.

Es versteht sich, dass die vorstehend genannten Zahlenwerte nur beispielhaft sind und keinesfalls eine Beschränkung der Erfindung beabsichtigen. Allerdings haben sich bei diesen Zahlenwerten besonders gute Ergebnisse gezeigt.

In Fig. 8 erkennt man noch ein auf den Schneidkopf 16 aufgesetztes Anbauteil 88, das der antriebsmäßigen Kopplung des Schneidkopfs 16 und der darin eingesetzten Schneidklingeneinheit 62 mit nicht näher dargestellten elektromotorischen Antriebsmitteln dient.

## Patentansprüche

1. Mikrochirurgisches Schneidinstrument (10) zur Erzeugung eines Hornhautflaps an einem Auge, umfassend
- eine auf das Auge (28) aufzusetzende, relativ zu diesem fixierbare Instrumentengrundkörpereinheit (12) und
- mindestens zwei Schneideinheiten (14,16) mit unterschiedlicher Schneidengeometrie, wobei eine erste (14) der Schneideinheiten geeignet ist, einen den Flaprand festlegenden Hornhautschnitt zu setzen, ohne dabei unter das Epithel zu fahren und dieses abzuheben, und eine zweite (16) der Schneideinheiten geeignet ist, in den durch die erste Schneideinheit (14) gesetzten Schnitt einzufahren und den Flap vom darunter liegenden Hornhautmaterial abzulösen, wobei der Instrumentengrundkörpereinheit Führungshaltemittel (30, 44) zugeordnet sind, welche dazu geeignet sind, die Schneideinheiten (14, 16) relativ zur Instrumentengrundkörperinheit beweglich geführt zu halten,
wobei die Führungshaltemittel (30, 44) dazu ausgebildet sind, die zweite Schneideinheit (16) so zu führen, dass sie in einen durch die erste Schneideinheit (14) gesetzten Flaprandschnitt einfährt und den Flap vom darunter liegenden Hornhautmaterial ablöst,
wobei die Führungshaltemittel (30, 44) mindestens eine erste Führungsformation (48) zur Führung der ersten Schneideinheit (14) und mindestens eine von der ersten Führungsformation verschiedene zweite Führungsformation (36, 46) zur Führung der zweiten Schneideinheit (16) umfassen, wobei die mindestens eine erste Führungsformation (48) an einem von der Instrunientengrundkörpereinheit (12) gesonderten, jedoch an dieser positionierbaren Führungskörper (44) ausgebildet ist, während die mindestens eine zweite Führungsformation (36, 46) an der Instrumentengrundkörpereinheit (12) ausgebildet ist, wobei für den Schneidbetrieb der zweiten Schneideinheit (16) der Führungskörper (44) von der Instrumentengrundkörpereinheit (12) abnehmbar ist**, dadurch gekennzeichnet, dass** die Führungshaltemittel (30, 44) die gleichzeitige Anordnung beider Schneideinheiten (14, 16) an der Instrumentengrundkörpereinheit (12) gestatten.

2. Schneidinstrument nach Anspruch 1,
**dadurch gekennzeichnet, dass** die erste Schneideinheit (14) eine Rundschneide (50) aufweist und die mindestens eine erste Führungsformation (48) zur rotatorischen Führung der ersten Schneideinheit (14) ausgebildet ist und dass die zweite Schneideinheit (16) eine geradlinige Schneide (66) aufweist und die mindestens eine zweite Führungsformation (36, 46) zur wenigstens näherungsweise linearen Führung der zweiten Schneideinheit (16) ausgebildet ist.

3. Schneidinstrument nach Anspruch 1 oder 2,
**dadurch gekennzeichnet, dass** der Führungskörper (44) eine näherungsweise zylindrische Führungsaufnahmeöffnung aufweist, in der die erste Schneideinheit (14) um die Zylinderachse drehbar aufnehmbar ist, und dass der Führungskörper (44) und die Instrumentengrundkörpereinheit (12) zusammenwitkende Verdrehsicherungsmittel (40, 42) aufweisen, welche den Führungskörper gegen Verdrehung relativ zur Instrumentengrundkörpereinheit (12) um die Zylinderachse sichern.

4. Schneidinstrument nach Anspruch 2 oder 3,
**dadurch gekennzeichnet, dass** die Schneide (66) der zweiten Schneideinheit (16) von einer flachen Schneidklinge gebildet ist, welche unter einem Winkel zwischen 18 und 32 Grad, vorzugsweise zwischen 21 und 28 Grad, höchstvorzugsweise von etwa 25 Grad zur Führungsrichtung der zweiten Schneideinheit (16) angeordnet ist.

5. Schneidinstrument nach einem der vorhergehenden Ansprüche,
**dadurch gekennzeichnet, dass** die zweite Schneideinheit (16) eine geradlinige, im Querschnitt rundliche Schneidkante (66) mit einem Rundungsradius zwischen 1 und 10 µm, vorzugsweise zwischen 3 und 8 µm, höchstvorzugsweise zwischen 4 und 6 µm besitzt und in einem unmittelbar an die Schneidkante (66) anschließenden ersten Schneidenbereich einen Schneidenwinkel zwischen 15 und 22 Grad, vorzugsweise zwischen 16 und 20 Grad, höchstvorzugsweise von etwa 18 Grad besitzt.

6. Schneidinstrument nach Anspruch 5,
**dadurch gekennzeichnet, dass** die zweite Schneideinheit (16) in einem an den ersten Schneidenbereich anschließenden zweiten Schneidenbereich einen Schneidenwinkel zwischen 10 und 18 Grad, vorzugsweise zwischen 11 und 16 Grad, höchstvorzugsweise von etwa 13 Grad besitzt, wobei der Schneidenwinkel des zweiten Schneidenbereichs kleiner als der Schneidenwinkel des ersten Schneidenbereichs ist, und dass gewünschtenfalls die zweite Schneideinheit in einem an den zweiten Schneidenbereich anschließenden dritten Schneidenbereich einen Schneidenwinkel zwischen 7 und 15 Grad, vorzugsweise zwischen 8 und 12 Grad, höchstvorzugsweise von etwa 9 Grad besitzt, wobei der Schneidenwinkel des dritten Schneidenbereichs kleiner als der Schneidenwinkel des zweiten Schneidenbereichs ist.

## Claims

1. A microsurgical cutting instrument (10) for generating a corneal flap on an eye, comprising
- an instrument base unit (12) to be placed on the eye (28), which can be fixed relative to the latter, and
- at least two cutting units (14, 16) with different cutting geometries,
wherein a first (14) of the cutting units is suited to make a corneal incision which defines the flap edge, without moving under the epithelium and lifting it off, and a second (16) of the cutting units is suited to move into the incision made by the first cutting unit (14) and to separate the flap from the corneal material which is located below,
wherein guide and support means (30, 44) are associated with the instrument base unit, which are suited to support the cutting units (14, 16) to be movably guided relative to the instrument base unit,
wherein the guide and support means (30, 44) are adapted to guide the second cutting unit (16) in such a manner that it moves into the flap edge incision made by the first cutting unit (14) and separates the flap from the corneal material which is located below,
wherein the guide and support means (30, 44) comprise at least one first guide formation (48) for guiding the first cutting unit (14) and at least one second guide formation (36, 46) different from the first guide formation, for guiding the second cutting unit (16),
wherein the at least one first guide formation (48) is formed at a guide body (44) separate from the instrument base unit (12) but that can be positioned on the latter, while the at least one second guide formation (36, 46) is formed at the instrument base unit (12),
wherein for the cutting operation of the second guide unit (16) the guide body (44) can be removed from the instrument base unit (12), **characterised in that**
the guide and support means (30, 44) permit simultaneous arrangement of both cutting units (14, 16) on the instrument base unit (12).

2. The cutting instrument according to claim 1, **characterised in that** the first cutting unit (14) has a circular cutting edge (50) and the at least one first guide formation (48) is designed for rotational guidance of the first cutting unit (14), and that the second cutting unit (16) comprises a rectilinear cutting edge (66) and the at least one second guide formation (36, 46) is designed for the at least approximately linear guidance of the second cutting unit (16).

3. The cutting instrument according to claim 1 or 2,
**characterised in that** the guide body (44) comprises an approximately cylindrical guide receptacle opening, in which the first cutting unit (14) can be accommodated so as to be rotatable about the cylindrical axis, and that the guide body (44) and the instrument base unit (12) comprise co-operating rotation prevention means (40, 42) which prevent the guide body from rotating about the cylindrical axis relative to the instrument base unit (12).

4. The cutting instrument according to claim 2 or 3,
**characterised in that** the cutting edge (66) of the second cutting unit (16) is formed by a flat cutting blade, which is arranged at an angle between 18° and 32°, preferably between 21° and 28°, and most preferably at an angle of about 25°, to the guiding direction of the second cutting unit (16).

5. The cutting instrument according to one of the previous claims,
**characterised in that** the second cutting unit (16) has a rectilinear cutting edge of rounded cross-section with a radius of curvature of between 1 and 10 µm, preferably between 3 and 8 µm, and most preferably between 4 and 6 µm, and in a first cutting region immediately adjoining the cutting edge (66) has a cutting angle between 15° and 22°, preferably between 16° and 20°, and most preferably of about 18°.

6. The cutting instrument according to claim 5,
**characterised in that** the second cutting unit (16) in a second cutting region adjoining the first cutting region has a cutting angle between 10° and 18°, preferably between 11° and 16°, and most preferably of about 13°, wherein the cutting angle of the second cutting region is less than the cutting angle of the first cutting region, and that, if desired, the second cutting unit in a third cutting region adjoining the second cutting region has a cutting angle between 7° and 15°, preferably between 8° and 12°, and most preferably of about 9°, wherein the cutting angle of the third cutting region is less than the cutting angle of the second cutting region.

## Revendications

1. Instrument coupant microchirurgical (10) pour soulever une fine lamelle (*flap*) à la surface de la cornée d'un oeil, comprenant
- une unité formant corps de base de l'instrument (12), destinée à être mise en place sur l'oeil (28) et à être fixée par rapport à celui-ci et
- au moins deux unités de coupe (14, 16) présentant une géométrie de coupe différente, une première (14) unité de coupe étant appropriée pour réaliser dans la cornée une incision déterminant le bord du flap sans pénétrer ce faisant sous l'épithélium et sans le soulever, et une deuxième (16) unité de coupe étant appropriée pour pénétrer dans l'incision réalisée par la première unité de coupe (14) et détacher le flap de la matière cornéenne sous-jacente,
des moyens de support de guidage (30, 44) étant associés à l'unité formant corps de base de l'instrument, lesquels sont appropriés pour maintenir les unités de coupe (14, 16) guidées de manière mobile par rapport à l'unité formant corps de base de l'instrument,
les moyens de support de guidage (30, 44) étant conçus pour guider la deuxième unité de coupe (16) de telle sorte qu'elle pénètre dans l'incision déterminant le bord du flap réalisée par la première unité de coupe (14) et détache le flap de la matière cornéenne sous-jacente,
les moyens de support de guidage (30, 44) comportant au moins une première formation de guidage (48) pour guider la première unité de coupe (14) et au moins une deuxième formation de guidage (36, 46) différente de la première formation de guidage pour guider la deuxième unité de coupe (16), ladite première formation de guidage (48) étant réalisée sur un corps de guidage (44) distinct de l'unité formant corps de base de l'instrument (12) mais cependant positionnable sur celle-ci, tandis que ladite deuxième formation de guidage (36, 46) est réalisée sur l'unité formant corps de base de l'instrument (12), ledit corps de guidage (44) pouvant être démonté de l'unité formant corps de base de l'instrument (12) pour la mise en service de la deuxième unité de coupe (16), **caractérisé en ce que** les moyens de support de guidage (30, 44) permettent le montage simultané des deux unités de coupe (14, 16) sur l'unité formant corps de base de l'instrument (12).

2. Instrument coupant selon la revendication 1,
**caractérisé en ce que** la première unité de coupe (14) présente une lame circulaire (50) et ladite première formation de guidage (48) est réalisée pour le guidage rotatoire de la première unité de coupe (14), et **en ce que** la deuxième unité de coupe (16) présente une lame rectiligne (66) et ladite deuxième formation de guidage (36, 46) est réalisée pour le guidage au moins approximativement linéaire de la deuxième unité de coupe (16).

3. Instrument coupant selon la revendication 1 ou 2,
**caractérisé en ce que** le corps de guidage (44) présente une ouverture de logement approximativement cylindrique dans laquelle la première unité de coupe (14) peut être logée mobile en rotation autour de l'axe cylindrique, et **en ce que** le corps de guidage (44) et l'unité formant corps de base de l'instrument (12) présentent des moyens de blocage en rotation (40, 42) coopérant entre eux, lesquels empêchent le corps de guidage de tourner autour de l'axe cylindrique, par rapport à l'unité formant corps de base de l'instrument (12).

4. Instrument coupant selon la revendication 2 ou 3,
**caractérisé en ce que** la lame (66) de la deuxième unité de coupe (16) est réalisée sous la forme d'une lame coupante plate, laquelle est disposée sous un angle compris entre 18 et 32 degrés, préférentiellement entre 21 et 28 degrés et encore plus préférentiellement d'environ 25 degrés par rapport à la direction de guidage de la deuxième unité de coupe (16).

5. Instrument coupant selon l'une des revendications précédentes,
**caractérisé en ce que** la deuxième unité de coupe (16) possède une arête coupante (66) rectiligne de section arrondie avec un rayon d'arrondi compris entre 1 et 10 µm, préférentiellement entre 3 et 8 µm, et encore plus préférentiellement entre 4 et 6 µm, et possède dans une première zone coupante directement adjacente à l'arête coupante (66) un angle de coupe compris entre 15 et 22 degrés, préférentiellement entre 16 et 20 degrés, et encore plus préférentiellement d'environ 18 degrés.

6. Instrument coupant selon la revendication 5,
**caractérisé en ce que** la deuxième unité de coupe (16) possède, dans une deuxième zone coupante adjacente à la première zone coupante, un angle de coupe compris entre 10 et 18 degrés, préférentiellement entre 11 et 16 degrés, et encore plus préférentiellement d'environ 13 degrés, l'angle de coupe de la deuxième zone coupante étant inférieur à l'angle de coupe de la première zone coupante, et **en ce que**, si on le souhaite, la deuxième unité de coupe possède, dans une troisième zone coupante adjacente à la deuxième zone coupante, un angle de coupe compris entre 7 et 15 degrés, préférentiellement entre 8 et 12 degrés, et encore plus préférentiellement d'environ 9 degrés, l'angle de coupe de la troisième zone coupante étant inférieur à l'angle de coupe de la deuxième zone coupante.
